# EUROPEAN PATENT SPECIFICATION

(11) **EP 1 945 648 B1**
(45) Date of publication and mention of the grant of the patent: **08.06.2011**
(21) Application number: 06809952.2
(22) Date of filing: 20.09.2006
(51) Int. Cl.: C07D 513/04, A61K 31/41, A61K 31/433, A61K 31/4196

(54) **DERIVATIVES OF 4,6-DISUBSTITUTED 1,2,4-TRIAZOLO-1,3,4-THIADIAZOLE, A PROCESS AND USES THEREOF**
DERIVATE VON 4,6-DISUBSTITUIERTEM 1,2,4-TRIAZOLO-1,3,4-THIADIAZOL, EIN VERFAHREN UND ANWENDUNGEN DAVON
DERIVÉS DE 4,6-BISUBSTITUE 1,2,4-TRIAZOLO-1,3,4-THIADIAZOLE, PROCEDÉ DE SYNTHÈSE DE CES DERNIERS ET UTILISATIONS CORRESPONDANTES

(30) Priority: 21.09.2005 IN CH13412005
(43) Date of publication of application: 23.07.2008
(73) Proprietor: Jawaharlal Nehru Centre for Advanced Scientific Research, Bangalore 560 064 Karnataka (IN); University of Mysore, Mysore 570 006 (IN)
(72) Inventor: KUNDU, Tapas, Kumar, Bangalore 560 064, Karnataka (IN); RANGAPPA, Kanchugarakoppal, Subbegowda, Karnataka (IN); SAILAJA, Badi, Sri, Andhra Pradesh (IN); VARIER, Radhika, Ashish, Coimbatore 641 012, Tamil Nadu (IN); SHIVANANJU, Nanjundaswamy, Karnataka (IN); BASAPPA,, Karnataka (IN)
(74) Representative: Shaw, Matthew Nigel
(86) International application number: PCT/IN2006/000379
(87) International publication number: WO 2007/034510

(56) References cited:
- US-A1- 2004 167 192
- SWAMY S.N. ET AL.: 'Synthesis of pharmaceutically important condensed heterocyclic 4,6-substituted-1,2,4-triazolo-1,3,4-thiadi azole derivatives as antimicrobials' EUR. J. MED. CHEM. vol. 41, no. 4, April 2006, pages 531 - 538, XP005410456
- MATHEW V., KESHAVAYYA J., VAIDYA V.P.: 'Heterocyclic system containing bridgehead nitrogen atom: synthesis and pharmacological activities of some substituted 1,2,4-triazolo[3,4-b]-1,3,4-thiadiazoles' EUR. J. MED. CHEM. vol. 41, no. 9, September 2006, pages 1048 - 1058, XP005651177
- LI D., LONG D., FU H.: 'Synthesis of 1,3-bis(3-aryltriazolo[3,4-b][1,3,4]thiadia zol-6-yl)benzenes' PHOSPHORUS, SULFUR AND SILICON AND THE RELATED ELEMENTS vol. 181, no. 3, 2006, pages 519 - 526, XP008078886
- INVIDIATA F.P. ET AL.: '3,6-Disubstituted 1,2,4-triazolo[3,4-b][1,3,4]thiadiazoles: synthesis, antimicrobial and antiviral activity. II' FARMACO vol. 51, no. 10, October 1996, pages 659 - 664, XP008078879
- INVIDIATA F.P. ET AL.: '3,6-Disubstituted 1,2,4-triazolo[3,4-b][1,3,4]thiadiazoles: synthesis and evaluation for antimicrobial and antiviral activity. III' FARMACO vol. 52, no. 4, April 1997, pages 259 - 261, XP008078878

## Description

### Field of the invention:

The present invention relates to the field of anti-neoplastic therapeutics and more particularly, the specific derivatives of 1, 2, 4-Triazolo-1, 3, 4-thiadiazole condensed heterocyclic nucleus bearing novel small molecules induce cancer-specific cell death. This invention also relates to process for the preparation of the novel compounds, pharmaceutical compositions containing the said compounds.

### Background of the Invention

Cancer is fundamentally a disease in which a population of cells cannot stop dividing. Often the dividing cells form a lump, or tumor; some cancers, such as leukemia, do not form tumors. Cancer of the uterine cervix remains one of the most common female malignancies in India. It accounts for about 26% of all female cancers and about 90,000 women are expected to develop the disease annually. The exact cause of cervical cancer is not known, but certain things like human papilloma virus (HPV) infection, sexual behaviour, contraceptive pill, unhealthy lifestyle, immune deficiency, and history of abnormal cells (dyskaryosis) appear to increase the risk. Changes in the cytogenetic equilibrium, such as chromosomal imbalances, allelic loss, and structural aberrations, happen during the transformation from normal epithelium to cervical cancer. Additional cofactors and mutational events may be important in the pathogenesis of invasive cervical cancers and may include chromosomal rearrangements, loss of constitutional heterozygosity, and proto-oncogene activation.

Oral and pharyngeal cancer is the sixth most common malignancy reported worldwide and one with high mortality ratios among all malignancies. The global number of new cases was estimated at 405,318 about two-thirds of them arising in developing countries. Highest rates are reported in South Asian countries such as India and Sri Lanka. The Indian sub-continent accounts for one-third of the world burden. The incidence and mortality from oral cancer is rising in several regions of Europe, Taiwan, Japan and Australia. In the USA alone, 30,000 Americans are diagnosed with oral or pharyngeal cancer each year. About 90 percent of head and neck cancers are of the squamous cell variety. Although there have been significant improvements in chemotherapy and surgical techniques, the disease is often particularly challenging to treat the secondary tumors.

Treatment options for cervical cancer include surgery, radiotherapy (Intensity Modulated Radiation Therapy (IMRT)) and chemotherapy (Intra-Arterial Chemotherapy). There are many factors that determine the type of treatment recommended. These include a woman's age and general health, as well as the exact type and stage of cancer. In early stages of the disease either surgery or radiotherapy, or a combination of both might be appropriate. For more advanced disease, radiotherapy is necessary, and may be used in combination with chemotherapy. Surgery, radiotherapy and chemotherapy can have side-effects, predominantly because of non- specific nature of the therapeutic agents or means. Here, we report the invention of a group of compounds, which preferably induce the cell death of squamous cancer cells, but not the normal cells or other cancer cells in culture.

This invention thus provides the novel compounds, which are lead for anti-neoplastic therapeutics. Further, it also provides a method for the preparation of the said compounds and pharmaceutical compositions containing the same. This invention particularly provides novel 6-(alkyl or aryl)-3-[1,2,4]triazolo[3,4-b][1,3,4]thiadiazole condensed heterocycles, their derivatives, their pharmaceutical acceptable solvates, pharmaceutical compositions containing them and their anti squamous cancer activities. More particularly, the present invention relates to the synthesis, complete characterization, their squamous cancer cell death induction and their pharmaceutically acceptable solvates and compositions containing them.

### Prior Art:

US Patent No: 7070965, entitled "Small molecule anticancer compounds and related production process" describes about a group of specific branched-chain fatty acids, with significant anticancer effects on human and animals; methods of making using either chemical synthesis or biosynthesis methods; and methods of treating cancer. However, the above document is not able to arrive at the application of the instant invention wherein it is able to arrive at absolutely novel and specific derivatives of 1,2,4-triazolo-1,3,4-thiadiazole with an ability to induce cervical and oral cancer cell death.

JP 55072188, entitled "1,3,4-thiadiazole derivative and its preparation" describes a process to arrive at 1,3,4-thiadiazole derivative of formula I [Py is 2-pyridyl, 2-(1-oxidopyridyl), 2-(5-lower alkylpyridyl)] in general and 2-Picolinoylamino-1,3,4-thiadiazole in specific. The derivatives were found to be effective against malignant tumor. However, the Japanese Patent was not able to arrive at the specific derivatives of 1,2,4-triazolo-1,3,4-thiadiazole which are effective and specific against cervical and oral cancer.

US Patent No: 6602873 entitled "Angiogenesis inhibiting thiadiazolyl pyridazine derivatives" wherein it describes about thiadiazolylpyridazine derivatives which are angiogenesis inhibitors-useful for treatment of diabetic retinopathy, osteo- and rheumatoid arthritis and solid tumours. However, the proposed methodology was not able to arrive at the application of the instant invention.

A research article entitled "Synthesis of pharmaceutically important condensed heterocyclic 4,6-disubstituted-1,2,4triazolo-1,3,4-thiadiazole derivatives as antimicrobials, S. Nanjunda Swamy, Basappa, B.S. Priya, B. Prabhuswamy, B.H. Doreswamy, J. Shashidhara Prasad, Kanchugarakoppal S. Rangappa, European Journal of Medicinal Chemistry, Volume 41, Issue 4, April 2006, Pages 531-538. This document is in relation to the derivatives of *1,2,4- triazolo-1,3.4-thiadiazole,* wherein it discloses all the possible substitutions at R₁ position and as regards the R₂ position. The above published article was not able to arrive at the derivatives of the application of the instant invention. Also, it is understood from the research article that the derivatives of 1, 2,4-triazole and 1,3,4-thiadiazole condensed nucleus system found to have diverse pharmacological activities in general and antitumor activity in particular. However, the research article was not able to establish activity for specific anti-cancer conditions such as cervical, breast or oral cancer. The applicant has arrived at novel derivatives of 4,6-disubstituted 1,2,4-triazolo-1,3,4-thiadiazole which are useful in cervical and oral cancer cells and does not show any toxicity on normal cells.

A research article entitled *"*New bis-aminomercaptotriazoles and bis-triazolothiadiazoles as possible anticancer agents, B. Shivarama Holla, K. Narayana Poojary, B. Sooryanarayana Rao, M.K. Shivananda, European Journal of Medicinal Chemistry, Volume 37, Issue6, June 2002, Pages 511-517. The instant article discloses anti cancer activity wherein it utilizes the following three cell lines, NCI-H 460 (Lung), MCF 7 (Breast) and SF 268 (Central Nervous System). However, from this article there is no motivation for the applicant to synthesize the derivatives of the application of the instant invention. Also, the article was not able to establish the specific anti-cancer activity as established by the applicant of the instant invention wherein the invention successfully establishes the same against cervical and oral cancer cell lines.

A research article entitled "Heterocyclic system containing bridgehead nitrogen atom: synthesis and pharmacological activities of some substituted 1,2,4-triazolo[3,4-b]-1,3,4-thiadiazoles" V. Mathew, J. Keshavayya, V.P. Vaidya, European Journal of Medicinal Chemistry, 2006. (Article in Press). The instant article establishes activity for the derivatives as antibacterial, antifungal, anti-inflammatory and analgesics. However, the article was not able to arrive at the application of the instant invention wherein it is able to arrive at the specific derivatives of 1,2,4-triazolo[3,4-b]-1,3,4-thiadiazoles which are effective against specific anti-cancerous conditions i.e. cervical and oral cancer.

A research article entitled "Synthesis and anticancer evaluation of some new hydrazone derivatives of 2,6-dimethylimidazo[2,1-b][1,3,4] thiadiazole -5-carbohydrazide" by Nalan Terzioglu and Aysel Gursoy, European Journal of Medicinal Chemistry, Volume 38, Issues 7-8 , July-August 2003, Pages 781-786. In this study the authors describes about novel 2,6-dimethyl-N'-substituted phenylmethylene-imidazo[2,1-b][1,3,4]thiadiazole-5-carbohydrazides (3a-3h) were synthesized from 2,6-dimethylimidazo-[2,1-b][1,3,4]thiadiazole-5-carbohydrazide (2) and were evaluated for anticancer activity against ovarian cancer cell line (OVCAR log10 GI50 value -5.51). The instant research article neither discloses the derivatives of instant invention nor the anti-cancerous activity against cervical and oral cancer cells.

A research article entitled "Synthesis and antimicrobial testing of 4H-1,2,4-triazole, 1,2,4-triazolo[3,4-b][1,3,4]thiadiazole and 1,2,4-triazolo[3,4-b][1,3,4]thiadiazine derivatives of 1H-benzimidazole" by Habib NS, Soliman R, Ashour FA, el-Taiebi M, published in the journal Pharmazie. Volume 52, November 1997, page number 844-847. The research article describes three novel series of benzimidazole derivatives. The prepared compounds were tested for antimicrobial activity in vitro; they showed moderate activity. It is understood from the research article that they have exclusively tried the activity of the derivatives for their antimicrobial purpose only and they have not tried against anti-cancer cell lines. Therefore, the research article was not able to arrive at the application of the instant invention.

A research article entitled "Aminothiadiazole (NSC #4728) in patients with advanced cervical carcinoma. A phase II study of the Gynecologic Oncology Group" by Asbury RF, Blessing JA, Mortel R, Homesley HD, Malfetano J, published in American Journal of Clinical Oncology 1987 Aug;10(4):299-301. The instant research article has evaluated twenty-one patients with advanced squamous-cell cervical cancer were treated with aminothiadiazole at a dosage of 125 mg/m2 weekly. Nineteen had prior chemotherapy. One patient had a partial response; six had stable disease; 14 had increasing disease; and two were unevaluable for response. There was a single life-threatening toxic episode. Aminothiadiazole used in this dosage and schedule has minimal activity in previously treated cervical carcinoma patients. However, the application of the instant invention is able to arrive at absolutely novel and specific derivatives of 1,2,4-triazolo-1,3,4-thiadiazole with an ability to induce cervical and oral cancer specific cell death with out inducing normal cell death.

None of the above patents and inventions, taken either singly or in combination, is seen to describe the instant invention as claimed. Accordingly, the instant invention was successful in arriving at the novel derivatives of 6-(alkyl or aryl)-3-[1,2,4]triazolo[3,4-b][1,3,4]thiadiazole condensed heterocycles, their derivatives, their pharmaceutical acceptable solvates and their anti squamous cancer activities and compositions containing them along with the pharmaceutically acceptable additives.

### Objects of the Invention:

The principal object of the present invention is to synthesize derivatives of 3,6-disubstituted 1,2,4-triazolo-1,3,4-thiadiazole.

Another object of the present invention is to develop a process for the synthesis of derivatives of 3,6-disubstituted-1,2,4-triazolo-1,3,4-thiadiazole.

Yet another object of the present invention is to characterize the derivatives of 3,6-disubstituted-1,2,4-triazolo-1,3,4-thiadiazole.

Still another object of the present invention is to induce death of squamous cancer cells but not normal cell death and other cancer cells in culture.

Still another disclosure of the present invention is to induce cervical cancer cell death.

Still another disclosure of the present invention is to induce oral or mouth cancer cell death.

### Statement of the Invention:

The present invention is in relation to compounds of 3,6-disubstituted-1,2,4-triazolo-1,3, 4-thiadiazole of formula I,

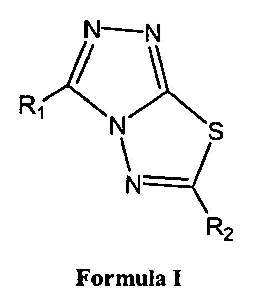

Wherein R₁ is selected from a group consisting of -CH₃, -CH₂-CH₃, -C₆H₅, -(4-Cl-C₆H₅), and -(4-CH₃-C₆H₅); and R₂ is selected from a group consisting of 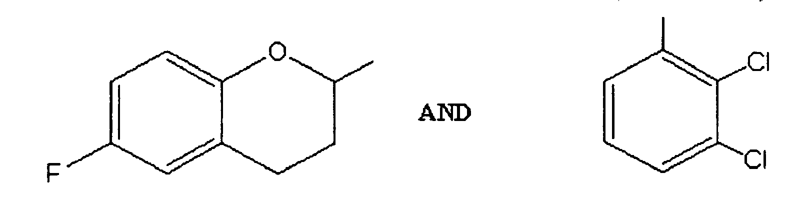 optionally along with pharmaceutically acceptable additives to form a pharmaceutical composition; a process for preparation of specific derivatives of 3,6-disubstituted 1,2, 4-triazolo-1,3,4-thiadiazole of fomula - I, 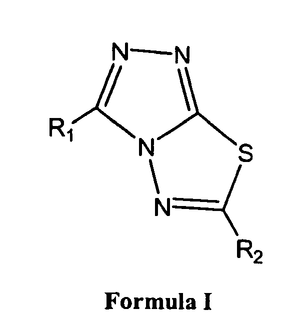 wherein, said process comprises steps of hydrazinolysation of aromatic esters, reaction of hydrazides with carbon disulfide in presence of alcoholic potassium hydroxide, condensation of potassium salt of thiocarbohydrazides, condensation and cyclisation of 3-aromatic or 3-aliphatic substituted-1,2,4-triazolo-5-thiols; and use of derivatives of 4,6-disubstituted 1,2,4- triazolo-1,3,4-thiadiazole of formula I,

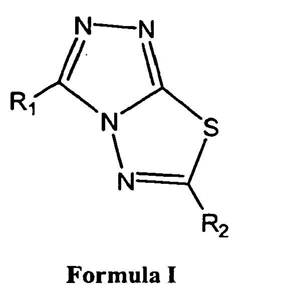

Wherein R₁ is selected from a group consisting of -CH₃, -CH₂-CH₃, -C₆H₅, - (4-Cl-C₆H₅), and -(4-CH₃-C₆H₅); and R₂ is selected from a group consisting of 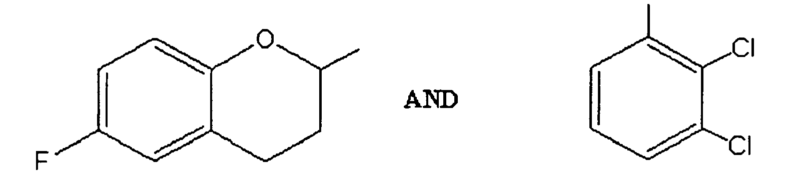 optionally along with pharmaceutically acceptable additives to form a pharmaceutical composition for manufacture of a medicament for anticancer therapy in a subject in need thereof, said method comprising administering pharmaceutically acceptable amount of the derivatives or the compositions to the subject. We have assigned the name TN series for the group A (6-fluorochroman-2-yl) substituted at R₂ position and the name NC series was assigned for the group B (2,3-dichlorophenyl) substituted at R₂ position.

### Brief description of the accompanying drawings

**Figure: 1** Shows comparison of extent of apoptosis induced by TN and NC series of compounds in cervical cancer, breast cancer and normal cells (**N.B**. TN and NC refers to different derivatives)
**Figure: 2** Shows comparison of extent of apoptosis induced by selected TN and NC series of compounds in different cancer cell lines.
**Figure: 3** Shows a flow cytometric quantitation of induction of apoptosis by cervical cancer specific compounds.
**Figure: 4** Shows schematic representation of synthesis of derivatives of 3, 6-disubstituted 1, 2, 4- triazolo-1, 3, 4-thiadiazole.

### Detailed description of the present invention

The embodiment of the present invention is in relation to derivatives of 3,6-disubstituted 1,2,4-triazolo-1,3,4-thiadiazole of formula I, 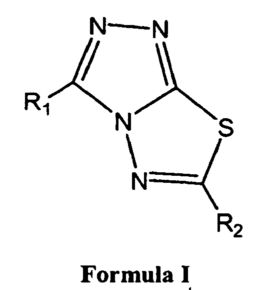 wherein R₁ is selected from a group consisting of -CH₃, -CH₂-CH₃, -C₆H₅, -(4-Cl-C₆H₅), and - (4-CH₃-C₆H₅); and R₂ is selected from a group consisting of 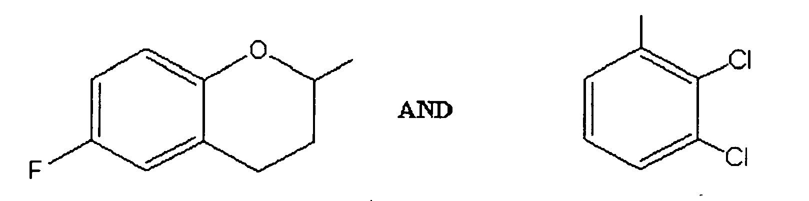 optionally along with pharmaceutically acceptable additives to form a pharmaceutical composition.

Another embodiment of the present invention, wherein said compounds are (6-(6-fluorochroman-2-yl)-3-phenyl-[1,2,4]triazolo[3,4-b][1,3,4]thiadiazole); (6-(6-fluorochroman-2-yl)-3-p-tolyl-[1,2,4]triazolo[3,4-b][1,3,4]thiadiazole); (6-(2,3-dichlorophenyl)-3-ethyl-[1,2,4]triazolo[3,4-b][1,3,4]thiadiazole); and (6-(2,3-dichlorophenyl)-3-(4-chlorophenyl)-[1,2,4]triazolo[3,4-b][1,3,4]thiadiazole.

In yet another embodiment of the present invention wherein the additives are selected from a group comprising granulating agents, binding agents, lubricating agents, disintegrating agents, sweetening agents, coloring agents, flavoring agents, coating agents, plasticizers, preservatives, suspending agents, emulsifying agents and spheronization agents.

The embodiment of the present invention is in relation to a process for preparation of specific compounds of 3,6-disubstituted 1,2,4- triazolo-1,3,4-thiadiazole of fomula- I, 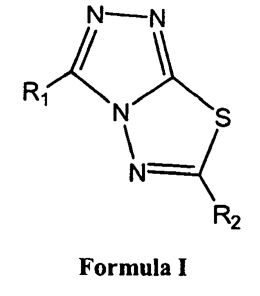 wherein, said process comprises steps of hydrazinolysation of aromatic esters, reaction of hydrazides with carbon disulfide in presence of alcoholic potassium hydroxide, condensation of potassium salt of thiocarbohydrazides, condensation and cyclisation of 3-aromatic or 3-aliphatic substituted- 1,2,4-triazolo-5-thiols.

Another embodiment of the present invention wherein the hydrazinolysation is carried using hydrazine.

In yet another embodiment of the present invention wherein said hydrazinolysation is carried for converting aromatic esters into corresponding hydrazides.

Still yet another embodiment of the present invention wherein the reaction of hydrazides with carbon disulfide in presence of alcoholic potassium hydroxide is carried for converting hydrazides into corresponding potassium salt of thiocarbohydrazide.

Still yet another embodiment of the present invention wherein said condensation is hydrazine hydrate added condensation of potassium salt of thiocarbohydrazide into corresponding 3-aromatic substituted-1,2,4-triazolo-5-thiols.

Still yet another embodiment of the present invention wherein the condensation of aliphatic acids with thiocarbohydrazide yields 3-aliphatic substituted- 1, 2, 4-triazolo-5-thiols.

Still yet another embodiment of the present invention wherein the cyclisation of 1, 2, 4-triazolo-5-thiols yields the compounds of formula I.

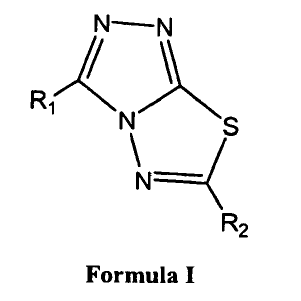

Still yet another embodiment of the present invention wherein said cyclisation carried using phosphorous oxychloride.

Still yet another embodiment of the present invention wherein said cyclisation is carried out at reflux temperature.

Still yet another embodiment of the present invention wherein said cyclisation reaction is carried out for time period ranging from 16 to 20 hrs.

Still yet another embodiment of the present invention wherein said cyclisation redaction is carried for about 18 hrs.

The embodiment of the present invention is in relation to use of compounds of 3,6-disubstituted 1,2,4-triazolo-1,3,4-thiadiazole of formula I, 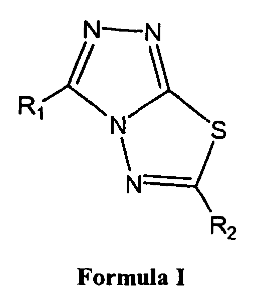 wherein R₁ is selected from a group consisting of -CH₃, -CH₂-CH₃, -C₆H₅, - (4-Cl-C₆H₅), and -(4-CH₃-C₆H₅); and R₂ is selected from a group consisting of 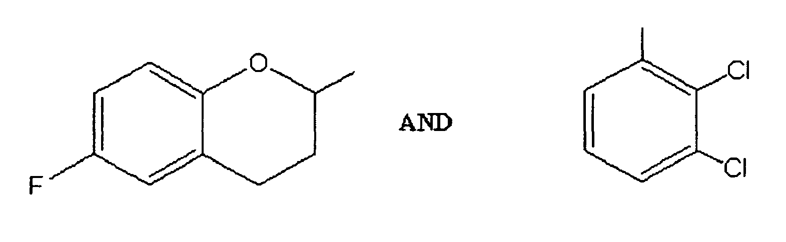 optionally along with pharmaceutically acceptable additives to form a pharmaceutical composition for manufacture of a medicament for anticancer therapy in a subject in need thereof, said method comprising administering pharmaceutically acceptable amount of the derivatives or the compositions to the subject.

Another embodiment of the present invention wherein the anticancer therapy is for cervical and oral cancers.

In yet another embodiment of the present invention wherein said derivatives are targeted anti-neoplastic agents.

Still yet another embodiment of the present invention wherein said derivatives induce cell death through apoptosis specifically to cervical and oral squamous cancer cells.

Still yet another embodiment of the present invention wherein said derivatives show no cellular toxicity for cell lines other than cervical and oral cancer cell lines.

Still yet another embodiment of the present invention wherein said derivatives activity is due to histone modifications.

Still yet another embodiment of the present invention wherein the subject is animal including human.

### Nature of Invention:

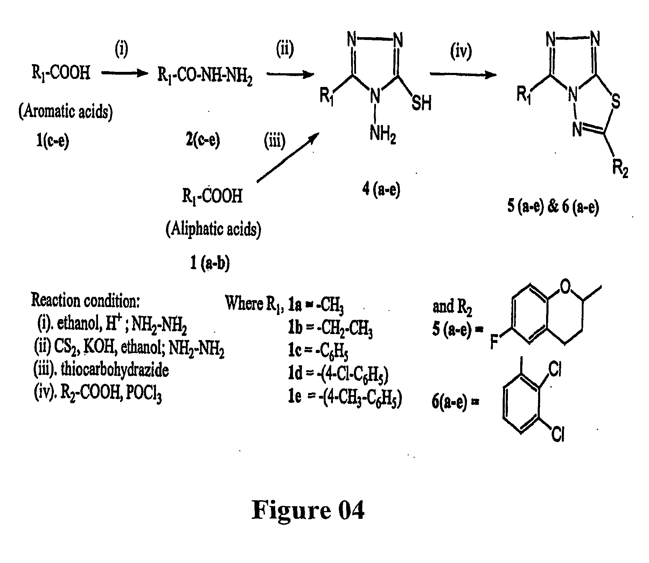

The bioactive or newer rings present at the 6^{th} and 3^{rd} position of the 1,2,4-Triazolo[4,5-b][1,3,4]thiadiazole nucleus bearing small molecules are emerging prominently as pharmaceutically important molecules because of their diverse effect on physiological pathways. These types of molecules demonstrated to possess anti-inflammatory, antitumor or antibacterial properties. The synthesis of bioactive/newer rings substituted title compounds involves the conversion of ester into their corresponding hydrazide. The reaction of hydrazides with carbon disulfide in presence of alcoholic KOH yield the potassium salt of thiocarbohydrazides followed by the hydrazine hydrate condensation reaction to obtain the substituted 1,2,4-triazolo-5-thiols. Similarly, the condensation of aliphatic acids with thiocarbohydrazide to yield the 3-aliphatic substituted-1,2,4-triazolo-5-thiols.The title compounds, **5a-e** and **6a-e** as shown in figure **4** were obtained by the condensation of 1,2,4-triazolo-thiols and different substituted acids by using POCl₃ as cyclising agent at reflux temperature for 18 hrs. Treating of different cancerous cell lines with these compounds was found to induce apoptosis specifically to squamous cancers. These compounds thus may serve as lead compounds to synthesize specific anti- neoplastic therapeutics.

The present invention relates to novel 6-(alkyl or aryl) 3-substituted-[1,2,4]triazolo[3,4-b][1,3,4]thiadiazole condensed heterocycles of the formula in according to the Figure: 4 of the accompanying drawings.

Having thus, described our invention and the manner and a process of making and using it in such a field, clear, concise and exact terms so as to enable any person skilled in the art to which it pertains or with which it is most nearly connected to make and use the same.
1. The title compounds 5a-e and 6a-e, having a general formula as shown in Figure: 4
2. A method for the preparation of specific derivatives of the compounds 5a-e and 6a-e as shown in figure: 4 of the present invention, which comprises efficient procedure for the large-scale synthesis and their crystal growth.
3. Discovery of the specific cancer, namely cervical and mouth (squamous) cell death of compounds having the general structure of the group consisting of 10 molecules and as defined by the structural formula as given in Figure-4.

The technology of the instant Application is further elaborated with the help of following examples. However, the examples should not be construed to limit the scope of the invention.

### Example 1

### Immunofluorescence

the cells were fixed in 4% paraformaldehyde in PBS (172 mM NaCl, 2.7 mM KCl, 8.1 mM Na₂HPO₄, and 1.76 mM KH₂PO₄) for 20 mins at room temperature. Subsequently, the cells were washed with PBS. The cells were treated with 0.1 µg/ml Hoechst 33258 for 30 minutes at 37 °C, rinsed with PBS and mounted them on slides with 70% glycerol. Stained nuclei were analysed by using 0.1 µg/ml Hoechst 33258 in PBS. Fluorescence for Hoechst were visualized by using different filters of the Carl Zeiss microscope (Axioskop 2 plus), the image was captured by AxioCam MRc camera and AxioVision 3.1 software was used, to process the images. Further confocal images were taken and % of apoptotic cells were determined.

### Example 2

### DNA Fragmentation assay

The TN and NC series induces apoptosis, which was monitored by the extent of DNA fragmentation. DNA was extracted from the untreated cells and TN, NC treated HeLa cells. The cells (3x 10⁶ per 90 mm dish) were seeded and treated with the compound for 24 hours. Harvested cells were washed with PBS and then lysed with lysis buffer containing 0.5% Triton X-100, 20 mM Tris and 15 mM EDTA at room temperarture for 15 minutes. The lysate was treated with Rnase (0.1 mg/ml) and proteinase K (2 mg/ml) for 1 h, extracted with phenol/chloroform /isoamyl alcohol (25:24:1) and DNA was precipated by incubating the upper aqueous phase with 0.1 volumes of 3 M sodium acetate (pH 5.2) and 1 volume of isopropanol overnight at -20 °C. The pellet obtained on centrifugation was washed with 70% ethanol and dissolved after airdrying in 50ul of TE buffer. The extracted DNA was analysed on 1.8% agarose gel and visualized by ethidium bromide staining.

### Example 3

### Cell Viability assay

Cell suspension of the cells to be assayed (about 10⁶ cells/ml) and 1:1 dilution of the suspension using a 0.4% trypan blue solution were prepared and loaded the counting chambers of a hemocytometer with the dilution. It was allowed to sit for 1-2 minutes and counting of the number of stained cells and total number of cells was done following the above procedure for Hemocytometer Counting. The calculated percentage of unstained cells represents the percentage of viable cells.

### Example 4

### Flow-cytometric analysis

Treated and untreated cells were washed twice in PBS and fixed for 30 minutes in 70 % ethanol (-20 °C). After repeated washing in PBS, the cells are stained with propidium iodide (50 µg/ml) and then treated with Rnase A (100 µg/ml). The cell cycle profile was detemined with a FACS Calibur flow cytometer (Becton Dickinson) equipped with argon-ion laser, using 488 nm laser line for excitation. Cell quest software running on an Apple Macintosh computer connected to flow cytometer was used for the data acquisition. The cell cycle phases were analysed with the aid of cell cycle software.

### Example 5

### Analysis of in vivo histones by SDS-Polyacrylamide gel electrophoresis

HeLa cells (3 x 10⁶ cells per 90-mm dish) were seeded overnight, and histones were extracted from 24 h of compound treated cells as reported previously. In brief, cells were harvested, washed in ice-cold buffer A (150 mM KCl, 20 mM HEPES, pH 7.9, 0.1 mM EDTA, and 2.5 mM MgCl₂) and lysed in buffer A containing 250 mM sucrose and 1% (v/v) Triton X-100. Nuclei were recovered by centrifugation and washed, and proteins were extracted for 1 h using 0.25 M HCl. The proteins were precipitated with 25% (w/v) trichloroacetic acid and sequentially washed with ice-cold acidified acetone (20 µl of 12 N HCl in 100 ml of acetone) and acetone, air-dried, and dissolved in the sample buffer (5.8 M urea, 0.9 M glacial acetic acid, 16% glycerol, and 4.8% 2-mercaptoethanol). The histones (equal amounts in all lanes) were resolved on SDS PAGE gel.

### Example 6

### Synthesis of the triazole derivatives

The synthesis of bioactive/newer rings substituted title compounds involves the conversion of substituted title compounds involves the conversion of substituted - aromatic esters into their corresponding hydrazides. The reaction of hydrazides with carbon disulpide in presence of alcoholic KOH to yield their corresponding potassium salt of thiocarbohydrazides followed by the hydrazine hydrate added condensation reaction to obtain the 3-aromatic substituted-1,2,4-triazolo-5-thiols.Similarly, the condensation of aliphatic acids with thiocarbohydrazide to yield the 3-aliphatic substituted-1, 2,4-triazolo-5-thiols. The title compounds were obtained by the condensation of 1,2,4-triazolo-thiols and different substituted acids by using POCl₃ as cyclising agent at reflux temperature for 18 hrs. The list of derivatives along with their IUPAC names is as give below wherein TN and NC refer to different derivatives.
**TN-1:** 6-(6-fluorochroman-2-yl)-3-methyl-[1,2,4]triazolo[3,4-b][1,3,4]thiadiazole
**TN-2:** 6-(6-fluorochroman-2-yl)-3-ethyl-[1,2,4]triazolo[3,4-b][1,3,4]thiadiazole
**TN-3:** 6-(6-fluorochroman-2-yl)-3-phenyl-[1,2,4]triazolo[3,4-b][1,3,4]thiadiazole
**TN-4:** 6-(6-fluorochroman-2-yl)-3-p-tolyl-[1,2,4]triazolo[3,4-b][1,3,4]thiadiazole
**TN-5:** 3-(4-chlorophenyl)- 6-(6-fluorochroman-2-yl)-[1,2,4]triazolo[3,4-b][1,3,4]thiadiazole
**NC-1:** 6-(2,3-dichlorophenyl)-3-methyl-[1,2,4]triazolo[3,4-b][1,3,4]thiadiazole
**NC-2:** 6-(2,3-dichlorophenyl)-3-ethyl-[1,2,4]triazolo[3,4-b][1,3,4]thiadiazole
**NC-3:** 6-(2,3-dichlorophenyl)-3-phenyl-[1,2,4]triazolo[3,4-b][1,3,4]thiadiazole
**NC-4:** 6-(2,3-dichlorophenyl)-3-p-tolyl-[1,2,4]triazolo[3,4-b][1,3,4]thiadiazole
**NC-5:** 6-(2,3-dichlorophenyl)-3-(4-chlorophenyl)-[1,2,4]triazolo[3,4-b][1,3,4]thiadiazole

### Example 7

### Results

### Comparison of extent of apoptosis induced by TN and NC series of compounds in cervical cancer, breast cancer and normal cancer -Immunoflourescence

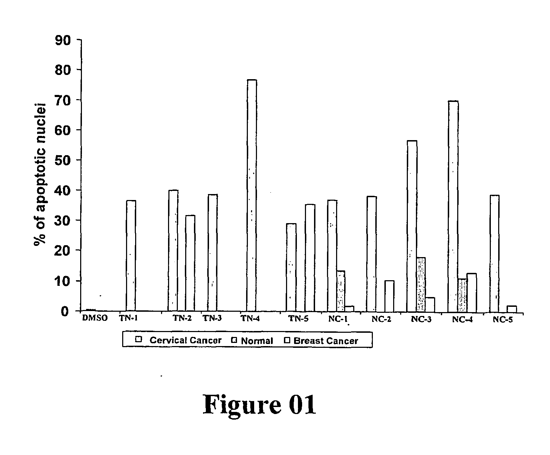

The 3 different cell lines were treated with TN-1, TN-2, TN-3, TN-4, TN-5, NC-1, NC-2, NC-3, NC-4 and NC-5. Most of the compounds showed apoptosis in cervical cancer, whereas in normal cell line, there is almost negligible apoptosis. In the Breast cancer some of the compounds were showing little apoptosis like TN-2 and TN-5 and remaining compounds showed negligible apoptosis. So compounds were selected in such a way that they are specific to cervical cancer cell line i.e specific to squamous cancer cell lines.
So finally TN-3, TN-4, NC-2 and NC-5 were selected. (Figure 1)

### Example 8

### Comparison of extent of apotosis induced by selected TN and NC series of compounds in different cancer cell lines-Immunoflourescence

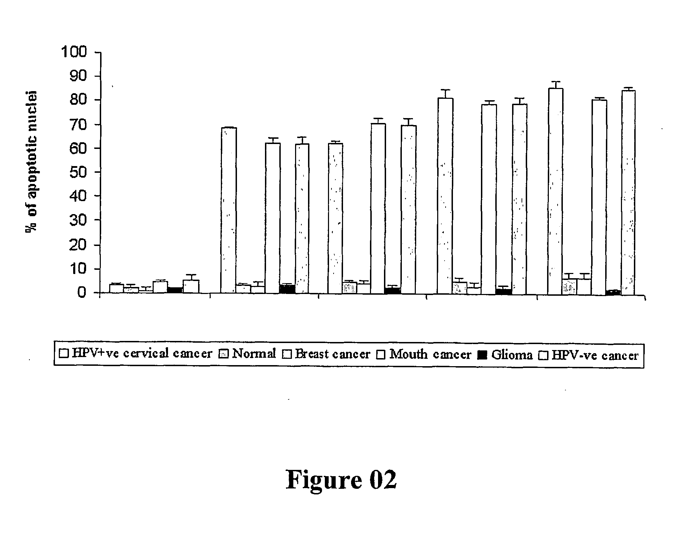

The different cancer cell lines, HeLa (Hpv +ve cancer), C33A (HPV -ve cancer), KB (Mouth cancer), MCF-7 (Breast cancer), 293T (Normal), and U373MG (Glioma) were treated with the aforementioned four selected compounds. Confocal analysis was done and % of apoptotic nuclei were plotted as graph. These compounds caused apoptosis in Cervical cancer cell lines and mouth cancer cell line, but not at all effective in Normal cancer cell line, Breast cancer cell line and Glioma saying that these compounds are very much specific to cervical cancer cell lines. (Figure 2)

### Example 9

### Induction of apoptosis by DNA Fragmentation Assay and Cell Viability Assay

The 4 compounds were very effectively causing apoptosis in cervical cancer cell lines and there was no apoptosis in case of normal cell line (293 T) and other cancer cell line (Hep G-2). Cell Viability assay was done to show the effect of these compounds on cervical cancer (HeLa). The % of apoptotic cells were determined and graph was plotted showing that these 4 compounds are very effective in causing apoptosis.

### Example 10

### Flow cytometric quantitation of induction of apoptosis by specific derivatives of triazolo-thiadiazole compounds

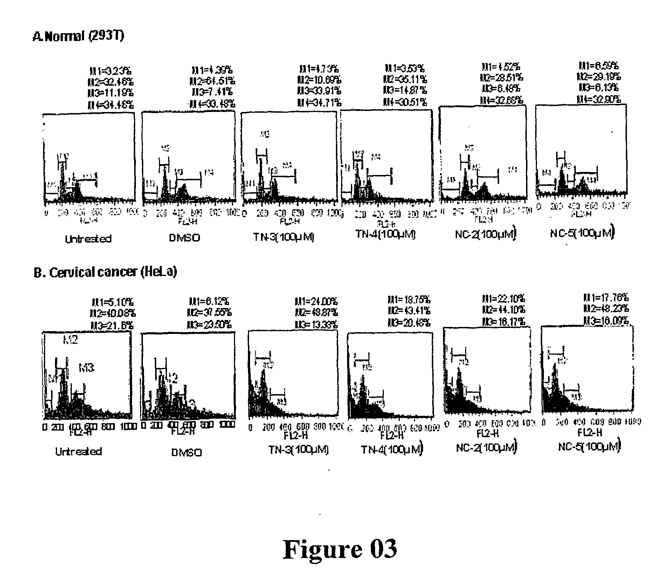

The cell cycle profile was determined with a FACS Calibur flow cytometer (Becton Dickinson). The subG1=M1 peak denote the apoptotic cells. Cervical cancer cells, when treated with these compounds undergo severe apoptosis. In normal cell line (293 T) the sub G1 population is too less indicating very less apoptosis The results clearly say that these compounds are not effective in other cell lines but very much specific to Squamous cancer cell lines (Figure 3).

### Example 11

### Effect of compounds on Histone modifications and gene regulation

The western blot analysis was done using the isolated histones and probed with H2AX antibody and the results say that 2 of the compounds (NC-2 and NC-5) get hyperphosphorylated. The Bax protein gets upregulated after these compound treatments on the cells and these compounds presumably causes apoptosis in p53 independent pathway as suggested from the unaltered p53 level upon compound treatment.

## Claims

1. A3. 6-disubstituted 1,2,4-triazolo-1, 3, 4-thiadiazole compound of formula I, 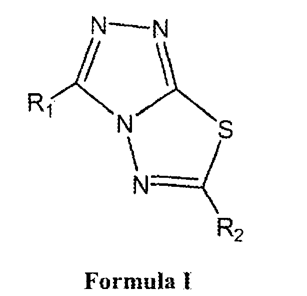 wherein R1 is selected from a group consisting of -CH3, -CH2-CH3, -C6H5, - (4-Cl-C6H5), and - (4-CH3-C6H5); and R2 is selected from a group consisting of 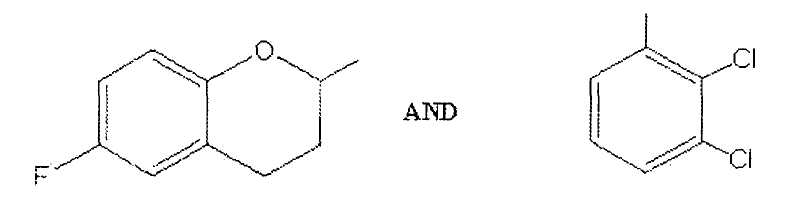 optionally along with pharmaceutically acceptable additives to form a pharmaceutical composition.

2. The compound as claimed in claim 1, wherein said compound is selected from a group consisting of (6-(6-fluorochroman-2-yl)-3-phenyl-[1,2,4]triazolo[3,4-b][1,3,4]thiadiazole); (6-(6-fluorochroman-2-yl)-3-p-tolyl-[1,2,4]triazolo[3,4-b][1,3,4]thiadiazole); (6-(2,3-dichlorophenyl)-3-etbyl-[1,2,4]triazolo[3,4-b][1,3,4]thiadiazole); and (6-(2,3-dichlorophenyl)-3-(4-chlorophenyl)-[1,2,4]triazolo [3,4-b][1,3,4]thiadiazole.

3. The compound as claimed in claim 1, wherein the additives are selected from a group comprising granulating agents, binding agents, lubricating agents, disintegrating agents, sweetening agents, coloring agents, flavoring agents, coating agents, plasticizers, preservatives, suspending agent, emulsifying agents and spheronization agents.

4. A process for the preparation of a 3, 6-disubstituted 1, 2, 4- triazolo-1, 3, 4-thiadiazole compound of formula - I, 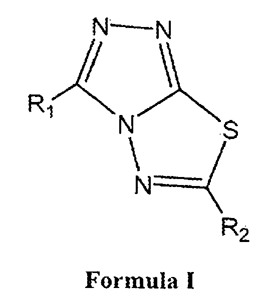 wherein, said process comprises steps of hydrazinolysation of aromatic esters, reaction of hydrazides with carbon disulfide in presence of alcoholic potassium hydroxide, condensation of potassium salt of thiocarbohydrazides, condensation and cyclisation of 3-aromatic or 3-aliphatic substituted- 1, 2, 4-triazolo-5-thiols.

5. The process as claimed in claim 4, wherein the hydrazinolysation is carried out using hydrazine.

6. The process as claimed in claim 4, wherein said hydrazinolysation is carried out for converting aromatic esters into corresponding hydrazides.

7. The process as claimed in claim 4, wherein the reaction of hydrazides with carbon disulfide in presence of alcoholic potassium hydroxide is carried out for converting hydrazides into corresponding potassium salt of thiocarbohydrazide.

8. The process as claimed in claim 4, wherein said condensation is hydrazine hydrate added condensation of potassium salt of thiocarbohydrazide into corresponding 3-aromatic substituted- 1,2,4-triazolo-5-thiols.

9. The process as claimed in claim 4, wherein the condensation of aliphatic acids with thiocarbohydrazide yields 3-aliphatic substituted- 1, 2, 4-triazolo-5-thiols.

10. The process as claimed in claim 4, wherein the cyclisation of 1, 2, 4-triazolo-5-thiols yields the compound of formula I.

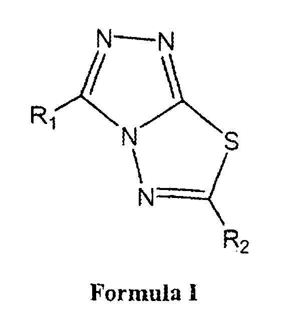

11. The process as claimed in claim 4, wherein said cyclisation carried out using phosphorous oxychloride.

12. The process as claimed in claim 4, wherein said cyclisation is carried out at reflux temperature.

13. The process as claimed in claim 4, wherein said cyclisation reaction is carried out for time period ranging from 16 to 20 hrs.

14. The process as claimed in claim 4, wherein said cyclisation reaction is carried out for about 18 hrs.

15. Use of a 3, 6-disubstituted 1, 2, 4- triazolo-1, 3, 4-thiadiazole compound of formula I, 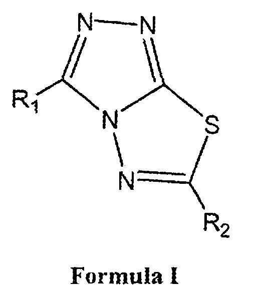 wherein R1 is selected from a group consisting of -CH₃, -CH₂-CH₃, -C₆H₅, -(4-Cl-C₆H₅), and -(4-CH₃-C₆H₅); and R₂ is selected from a group consisting of 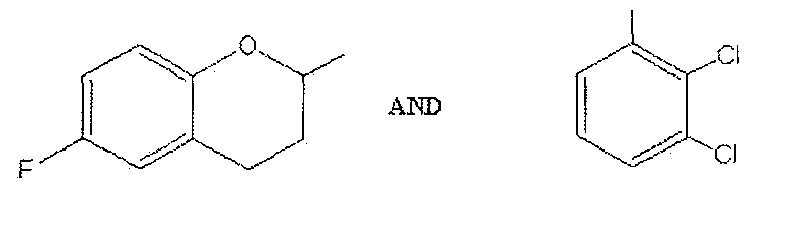 optionally along with pharmaceutically acceptable additives to form a pharmaceutical composition for manufacture of a medicament for anticancer therapy in a subject in need thereof, said method comprising administering pharmaceutically acceptable amount of the compound or the compositions to the subject.

16. Use as claimed in claim 15, wherein the anticancer therapy is for cervical and oral cancers.

17. Use as claimed in claim 15, wherein said compound is a targeted anti-neoplastic agent.

18. Use as claimed in claim 15, wherein said compound induces cell death through apoptosis specifically to cervical and oral squamous cancer cells.

19. Use as claimed in claim 15, wherein said compound's activity is due to histone modifications.

20. Use as claimed in claim 15, wherein the subject is animal including human.

## Patentansprüche

1. 3,6-disubstituierte 1,2,4-Triazol-1,3,4-thiadiazolverbindung der Formel 1, 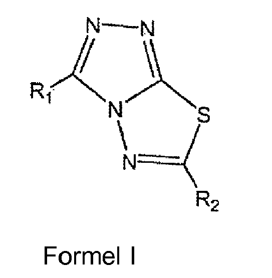 wobei R₁ ausgewählt ist aus einer Gruppe, bestehend aus -CH₃, -CH₂-CH₃, -C₆H₅, -(4-Cl-C₆H₅) und -(4-CH₃-C₆H₅), und R₂ ausgewählt ist aus einer Gruppe, bestehend aus 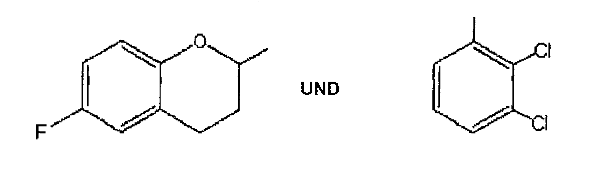 gegebenenfalls zusammen mit pharmazeutisch annehmbaren Zusätzen zur Bildung eines pharmazeutischen Präparats.

2. Verbindung nach Anspruch 1, wobei die Verbindung ausgewählt ist aus einer Gruppe, bestehend aus (6-(6-Fluorochroman-2-yl)-3-phenyl-[1,2,4]-triazol-[3,4-b]-[1,3,4]-thiadiazol); (6-(6-Fluorochroman-2-yl)-3-p-tolyl-[1,2,4]-triazol-[3,4-b]-[1,3,4]-thiadiazol); (6-(2,3-Dichlorphenyl)-3-ethyl-[1,2,4]-triazol-[3,4-b]-[1,3,4']-thiadiazol); und (6-(2,3-Dichlorphenyl)-3-(4-chlorphenyl)-[1,2,4]-triazol-[3,4-b]-[1,3,4]-thiadiazol.

3. Verbindung nach Anspruch 1, wobei die Zusätze aus einer Gruppe ausgewählt sind, die Granulierungsmittel, Bindemittel, Schmiermittel, Desintegrationsmittel, Süßstoffe, Farbstoffe, Aromastoffe, Beschichtungsmittel, Weichmacher, Konservierungsmittel, Suspensionsmittel, Emulgatoren und Sphäronisierungsmittel umfasst.

4. Verfahren zur Herstellung einer 3,6-disubstituierten 1,2,4-Triazol-1,3,4-thiadiazolverbindung der Formel 1, 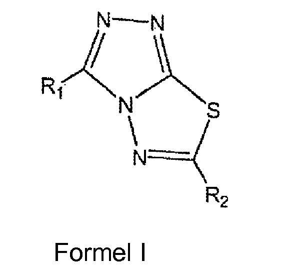 wobei das Verfahren Schritte zur Hydrazinolysierung aromatischer Ester, Reaktion von Hydraziden mit Kohlendisulfid in Gegenwart von alkoholischem Kaliumhydroxid, Kondensation von Kaliumsalz von Thiocarbohydraziden, Kondensation und Zyklisierung von 3-aromatischen oder 3-aliphatischen substituierten 1,2,4-Triazol-5-thiolen umfasst.

5. Verfahren nach Anspruch 4, wobei die Hydrazinolysierung unter Verwendung von Hydrazin ausgeführt wird.

6. Verfahren nach Anspruch 4, wobei die Hydrazinolysierung zum Umwandeln aromatischer Ester in entsprechende Hydrazide ausgeführt wird.

7. Verfahren nach Anspruch 4, wobei die Reaktion von Hydraziden mit Kohlendisulfid in Gegenwart von alkoholischem Kaliumhydroxid zum Umwandeln von Hydraziden in entsprechendes Kaliumsalz von Thiocarbohydrazid ausgeführt wird.

8. Verfahren nach Anspruch 4, wobei die Kondensation eine Kondensation von Kaliumsalz von Thiocarbohydrazid unter Zugabe von Hydrazinhydrat in entsprechende 3-aromatische substituierte 1,2,4-Triazol-5-thiole ist.

9. Verfahren nach Anspruch 4, wobei die Kondensation aliphatischer Säuren mit Thiocarbohydrazid 3-aliphatische substituierte 1,2,4-Triazol-5-thiole ergibt.

10. Verfahren nach Anspruch 4, wobei die Zyklisierung von 1,2,4-Triazol-5-thiolen die Verbindung der Formel I ergibt.

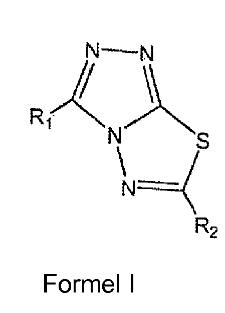

11. Verfahren nach Anspruch 4, wobei die Zyklisierung unter Verwendung von Phosphoroxychlorid ausgeführt wird.

12. Verfahren nach Anspruch 4, wobei die Zyklisierung bei Rückflusstemperatur ausgeführt wird.

13. Verfahren nach Anspruch 4, wobei die Zyklisierungsreaktion eine Zeitdauer lang im Bereich von 16 bis 20 Stunden ausgeführt wird.

14. Verfahren nach Anspruch 4, wobei die Zyklisierungsreaktion ungefähr 18 Stunden lang ausgeführt wird.

15. Verwendung einer 3,6-disubstituierten 1,2,4-Triazol-1,3,4-thiadiazolverbindung der Formel I, 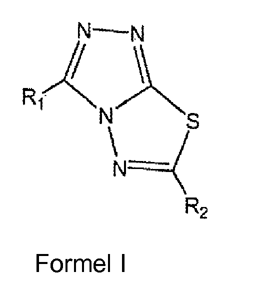 wobei R₁ ausgewählt ist aus einer Gruppe, bestehend aus -CH₃, -CH₂-CH₃, -C₆H₅, -(4-Cl-C₆H₅) und - (4-CH₃-C₆H₅), und R₂ ausgewählt ist aus einer Gruppe, bestehend aus 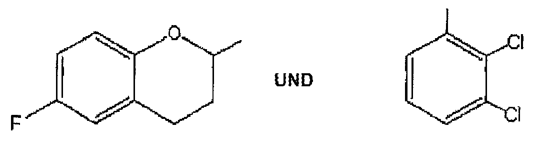 gegebenenfalls zusammen mit pharmazeutisch annehmbaren Zusätzen zur Bildung eines pharmazeutischen Präparats zur Herstellung eines Medikaments zur Antikrebstherapie bei einem Patienten, der dessen bedarf, wobei das Verfahren die Verabreichung einer pharmazeutisch annehmbaren Menge der Verbindung oder der Präparate an den Patienten umfasst.

16. Verwendung nach Anspruch 15, wobei die Antikrebstherapie für Gebärmutterhals- und Mundkrebs ist.

17. Verwendung nach Anspruch 15, wobei die Verbindung ein zielgerichtetes Antikrebsmittel ist.

18. Verwendung nach Anspruch 15, wobei die Verbindung Zelltod durch Apoptose speziell bei Gebärmutterhals- und Mund-Epithelkrebszellen induziert.

19. Verwendung nach Anspruch 15, wobei die Aktivität der Verbindung aufgrund von Histonmodifikationen erfolgt.

20. Verwendung nach Anspruch 15, wobei der Patient ein Tier einschließlich des Menschen ist.

## Revendications

1. Un composé de 3,6-bisubstitué 1, 2, 4- triazolo-1, 3, 4-thiadiazole de formule 1, 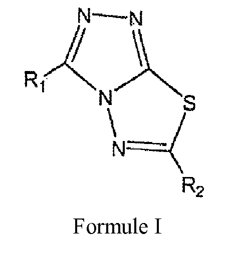 où R1 est sélectionné dans un groupe composé de -CH3, -CH2-CH3, -C6H5, - (4-C1-C6H5), et - (4-CH3-C6H5) ; et R2 est sélectionné dans un group composé de 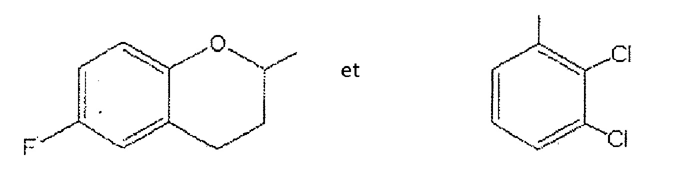 et, facultativement, d'additifs pharmaceutiquement acceptables pour former une composition pharmaceutique.

2. Le composé de la revendication 1, dans lequel ledit composé est sélectionné dans un groupe composé de
(6-(6-fluorochroman-2-yl)-3-phényl-[1,2,4]triazolo[3,4-b][1,3,4]thiadiazole);
(6-(6-fluorochroman-2-yl)-3-p-tolyl-[1,2,4]triazole [3,4-b][1,3,4] thiadiazole);
(6-(2,3-dichlorophényl)-3-éthyl-[1,2,4]triazole[3,4 b][1,3,4']thiadiazole) ; et (6-(2,3-dichlorophényl)-3-(4-chlorophényl)-[1,2,4]triazolo[3,4-b][1,3,4]thiadiazole.

3. Le composé de la revendication 1, dans lequel les additifs sont sélectionnés dans un groupe composé d'agglutinants, de liants, de lubrifiants, de désintégrants, d'édulcorants, de colorants, d'aromatisants, d'agents enrobants, d'agents plastifiants, de conservateurs, d'agents de suspension, d'émulsifiants et d'agents de sphéronisation.

4. Un procédé de préparation d'un composé de 3, 6-bisubstitué 1, 2, 4- triazolo-1, 3, 4-thiadiazole de formule 1, 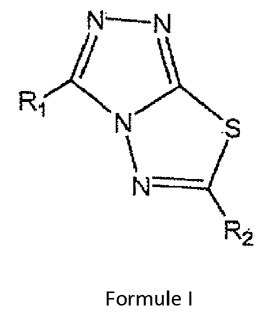 ledit procédé comprenant des étapes d'hydrazinolyse d'esters aromatiques, de réaction des hydrazides avec le sulfure de carbone en présence d'hydroxyde de potassium alcoolique, la condensation de sel de potassium de thiocarbohydrazides, la condensation et la cyclisation de 3-aromatique ou 3-aliphatique substitué - 1, 2, 4-triazolo-5-thiols.

5. Le procédé selon la revendication 4, dans lequel une hydrazinolyse est réalisée au moyen d'hydrazine.

6. Le procédé selon la revendication 4, dans lequel ladite hydrazinolyse est réalisée afin de transformer les esters aromatiques en hydrazides correspondants.

7. Le procédé selon la revendication 4, dans lequel la réaction des hydrazides avec le sulfure de carbone en présence d'hydroxyde de potassium alcoolique est réalisée afin de transformer les hydrazides en sel de potassium de thiocarbohydrazide correspondant.

8. Le procédé selon la revendication 4, dans lequel ladite condensation est la condensation avec l'hydrate d'hydrazine de sel de potassium de thiocarbohydrazide en 3-aromatique substitué- 1,2,4-triazolo-5-thiols correspondants.

9. Le procédé selon la revendication 4, dans lequel la condensation d'acides aliphatiques avec du thiocarbohydrazide donne des 3-aliphatique substitué-1,2, 4-triazolo-5-thiols.

10. Le procédé selon la revendication 4, dans lequel la cyclisation de 1, 2, 4-triazolo-5-thiols donne le composé de formule I.

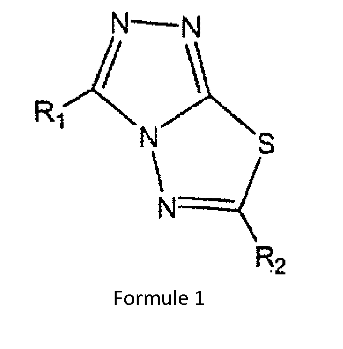

11. Le procédé selon la revendication 4, dans lequel ladite cyclisation est réalisée au moyen d'oxychlorure de phosphore.

12. Le procédé selon la revendication 4, dans lequel ladite cyclisation est réalisée à température de reflux.

13. Le procédé selon la revendication 4, dans lequel ladite réaction de cyclisation est réalisée sur une période de 16 à 20 heures.

14. Le procédé selon la revendication 4, dans lequel ladite réaction de cyclisation est réalisée pendant environ 18 heures.

15. L'utilisation d'un composé de 3, 6-bisubstitué 1, 2, 4- triazolo-1, 3, 4-thiadiazole de formule 1 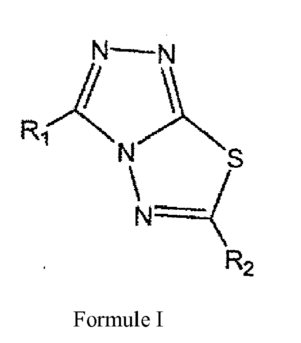 dans lequel R1 est sélectionné dans un groupe composé de -CH₃, -CH₂-CH₃, -C₆H₅, -(4-Cl-C₆H₅), et -(4-CH₃-C₆H₅); et R₂ est sélectionné dans un groupe composé de 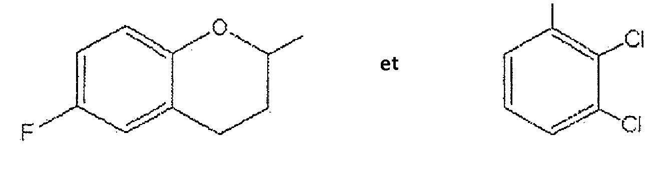 facultativement, d'additifs pharmaceutiquement acceptables pour former une composition pharmaceutique dans le but de fabriquer un médicament utilisable dans le cadre du traitement anticancéreux d'un sujet en ayant besoin, ladite méthode comprenant l'administration d'une dose pharmaceutiquement acceptable du composé ou des compositions audit sujet.

16. L'utilisation selon la revendication 15, **caractérisée en ce que** ledit traitement anticancéreux cible les cancers du col de l'utérus et de la bouche.

17. L'utilisation selon la revendication 15 **caractérisée en ce que** ledit composé est un agent antinéoplasique ciblé.

18. L'utilisation selon la revendication 15, **caractérisée en ce que** ledit composé induit la mort cellulaire par apoptose spécifiquement des cellules squameuses cancéreuses du col de l'utérus et de la bouche.

19. L'utilisation selon la revendication 15, **caractérisée en ce que** l'activité du composé résulte de la modification d'histones.

20. L'utilisation selon la revendication 15 **caractérisée en ce que** le sujet est un animal, y compris l'être humain.
